# EUROPEAN PATENT SPECIFICATION

(11) **EP 2 313 057 B1**
(45) Date of publication and mention of the grant of the patent: **07.08.2019**
(21) Application number: 09769330.3
(22) Date of filing: 25.06.2009
(51) Int. Cl.: A61K 8/06, A61K 8/20, A61K 8/73, A61Q 19/06

(54) **COSMETIC COMPOSITION, USE AND METHOD OF PREPARATION**
KOSMETISCHE ZUSAMMENSETZUNG, VERWENDUNG UND VERFAHREN ZUR HERSTELLUNG
COMPOSITION COSMÉTIQUE, UTILISATION ET PROCÉDÉ DE PRODUCTION ASSOCIÉ

(30) Priority: 26.06.2008 IT UD20080153
(43) Date of publication of application: 27.04.2011
(73) Proprietor: Biofarma S.p.A., 33036 Mereto di Tomba (IT)
(72) Inventor: SCARPA, Germano, I-33100 Udine (IT)
(74) Representative: Petraz, Gilberto Luigi
(86) International application number: PCT/EP2009/057995
(87) International publication number: WO 2009/156482

(56) References cited:
- EP-A- 1 477 159
- WO-A-2004/060393
- WO-A-2004/069217
- DE-A1- 10 305 965
- US-A- 5 571 503
- US-A1- 2007 231 279

## Description

### FIELD OF THE INVENTION

The present invention concerns a cosmetic composition in the form of a saline emulsion, of the type applicable on the skin, to give a non-restrictive example for use as an anti-swelling product, in order to drain the excess cutaneous water through an osmotic effect, and as an anti-cellulite.

### BACKGROUND OF THE INVENTION

It is known to use oil in water emulsions as cosmetic compositions to spread on, such as cream, or to apply; these are emulsions in which the water component is dispersed in the fatty component.

The fatty component, because of its affinity with the skin, promotes the applicability of the composition.

Typically, an oil in water emulsion tends, in itself, to be unstable and is stabilized by using emulsion stabilizers. Otherwise, the emulsion would break down, or rather the water component would separate from the fatty component. In practical terms this would cause, for the final consumer, a not very pleasant appearance and sensation on the skin, resulting in a limited commercial success of a product of the type in question, as well as a limited efficiency.

Furthermore, it is known that an inorganic solubilized salt can be used to "deflate" those parts of the body on which it is applied, eliminating the accumulated cutaneous liquids, thanks to its osmotic and draining effect. It can happen that a determinate amount of water tends to accumulate in the extra-cellular interstices of the skin, with consequent un-aesthetic effects and water retention. This phenomenon is often linked to the onset of cellulite.

In the state of the art, the addition of a salt to an emulsion, even if desirable, encounters many difficulties, inasmuch as a high quantity of salt can cause the breakdown of the emulsion, with the consequences described above. On the other hand a reduced quantity of salt, to prevent the breakdown of the emulsion, may not be sufficient for an efficacious deflating and draining effect.

Prior art documents WO-A-2004/069217, US-A-2007/231279, EP-A-1.447.159, DE-103.05.965, US-A-5,571,503 and WO-A-2004/060393 disclose oil in water emulsions in which the content of salt is low, less than 5% in weight.

Purpose of the present invention is to produce a cosmetic composition in the form of an oil in water emulsion and to perfect a method for its production, with an efficacious and high deflating and draining effect which, at the same time, is stable, particularly when it is used by the final consumer.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purpose, a cosmetic composition, in the form of an oil in water saline emulsion, according to the present invention comprises a fatty part and water.

According to a first feature, the cosmetic composition of the present invention comprises a determinate quantity of a viscous-making product able to reticulate the water and a determinate quantity of sodium chloride.

According to a variant solution of the present invention, the percentage in weight of the salt is comprised of between 9% and 30%, preferably between about 9% and 25%.

According to a preferred form of embodiment, the present invention provides that the cosmetic composition comprises the salt in a percentage of weight comprised between about 9% and 16%, more preferably between about 10% and 15%, even more preferably between about 11% and 14%.

The present invention allows to obtain a product for treating cellulite in the form of an oil in water saline emulsion with a great draining capacity, thanks to the high content of salt as described above and which, at the same time, has optimum stability of the emulsion, despite the aforesaid great saline percentage present which normally would tend to break down the emulsion. This balance between the two requirements, draining effect and stability of the emulsion, is advantageously obtained thanks to the combination and synergic action of the components that sequester the water present in the cosmetic composition, that is, the salt, the fatty part and the viscous-making product able to reticulate the water.

For the present invention therefore, normal table salt used in foods can be used (NaCl).

Sodium Chloride (NaCl) has a greater draining effect than other inorganic salts such as chlorinates or sulfates, having as a cation sodium, potassium, magnesium or aluminum, and therefore it is more effective to use them in the cosmetic composition under discussion for the treatment of cellulite. This is because the negative ion Cl⁻ involved has smaller sizes than the other negative ions mentioned above, and therefore, given the same weight of the inorganic salt, we have a greater draining effect using chlorides, for example sodium chloride.

Salt, particularly in the preferred high percentages as set forth above, allows to obtain a more pleasant texture, or rather consistency, of the final cosmetic composition, giving a product which is not sticky and can be easily absorbed, together with an efficacious draining and anti-cellulite effect.

The viscous-making product able to reticulate water is advantageously comprised in a percentage between about 1.5% and 3%, preferably between 1.8% and 2.7%, even more preferably between 1.9% and 2.5%, and allows the emulsion to stabilize despite the high percentage in weight of salt.

Advantageously, the viscous-making product determines a lattice in the water, which retains the saline solution formed by the water and salt. The water which remains free is then available for emulsion with the fatty part. In this way it is certain that the emulsion is rendered stable, because, despite the high percentage of salt, it is trapped in the lattice.

The viscous-making product is chosen from xanthan gums or hydroxyethyl- cellulose.

In particular, xanthan gum is a component of the class of gums, hydrophilic colloids and derivatives, and acts as a viscous-maker for water, ligand and stabilizer. A similar effect can be obtained with hydroxyethyl-cellulose.

The family of xanthan gums have the advantage that they are very efficient at trapping salt, as described above, but without breaking the lattice, which happens on the contrary with other gelatinizing/reticulating hydrocolloids, such as carbomer or guar gum, which are more sensitive to salt, and in which there is a break in the lattice and huge problems of reduced texture in the final product.

Advantageously, the quantity of water used varies between about 50% and 70% in weight, preferably between 54% and 68%. An advantageous solution of the present invention provides to use de-mineralized water to make up the cosmetic composition. However, ordinary water from the water system can also be used.

Advantageously, de-mineralized water has an electric conductivity less than 5 µS/cm (for example it can be in the range between about 1 µS/cm and 5 µS/cm).

De-mineralized water has the advantage of reducing or eliminating the contribution of unwanted amounts of salt, even if only minimal, as well as the salt deliberately used to produce the cosmetic composition according to the invention. In fact, since there is already a high content of inorganic salt, even small unexpected oscillations in the saline contribution of the water could cause a breakdown of the emulsion.

In this way, an even greater guarantee of the stability of the final emulsion is obtained. Therefore, during the production step there will always be a constant saline contribution, which does not depend on the supply of mains water. Therefore the saline parameter is made constant, without unwanted fluctuations. This is reflected in the high quality and homogeneity of the final characteristics of the cosmetic composition produced.

According to a variant, the de-mineralized water comes from a demineralization plant using inverse osmosis.

Advantageously, the inverse osmosis plant is able to guarantee a purity of de-mineralized water of a pharmaceutical level, that is, it uses a de-mineralized water with characteristics suitable for pharmaceutical production, which is known to have stricter parameters of purity. This guarantees a drastic reduction in pollutants in the water produced. The de-mineralized water is advantageously disinfected.

The fatty part of the cosmetic composition according to the present invention is typically comprised between about 6% and 12% in weight, preferably between 7% and 9% and this percentage can be given by different amounts of different fatty materials. The main function of the fatty part is to facilitate the application and to tone down the "texturizing" components of the final product, which otherwise would be like a sticky saline geloid.

According to an advantageous variant, the saline emulsion according to the present invention includes clay, preferably in a percentage between about 1% and 3%, even more preferably between about 1.5% and 2.5%. The presence of clay allows to obtain a saline emulsion which is like an applied product rather than a cream: in this way the cosmetic composition according to the present invention is for more professional use.

The cosmetic composition according to the present invention therefore provides an optimum balance between three principle components that sequester water, that is, the salt, the fatty part and the viscous-making product able to reticulate the water.

According to an advantageous variant solution, the cosmetic composition according to the present invention can also contain particular preserving compounds, in a composition comprised between about 1% and 2% (in this case too the overall amount can be given by the sum of the percentage of different preservatives) to slow down the deterioration of the final product. For instance, it is possible to use compounds of the ester-phenol group, such as parabens and/or alcohol-ethers, such as phenoxyethanol.

According to another formulation, the cosmetic composition can contain other stabilizing and viscous-making compounds, chosen from the class of fatty alcohols, for example cetearyl alcohol, or acarboxyl alcohol silated acids and PEG-20 stearate, in a percentage comprised between about 4% and 8%, preferably between about 4.5% and 7.5%.

Furthermore, according to needs, it is also possible to add moisturizing functional compounds, occlusive and protective, of the siloxane and xylane groups, in a percentage in weight comprised between about 1.5% and 3.5%, preferably between about 2% and 3%. Typically, perfumes can also be added to the cosmetic composition.

A preferred formulation of the saline emulsion according to the invention, which has been shown both to have an optimum stability of the emulsion, a good storability over time, a good texture, and also to offer considerable improvements in the draining effect of the water retained in the extra-cellular interstices and in reducing the phenomenon of cellulite, comprises:
- de-mineralized water comprised between about 54% and 68% in weight;
- viscous-making product able to reticulate the water comprised between about 1.5% and 3% in weight;
- sodium chloride comprised between about 10% and 15% in weight;
- fatty part in composition of about 8% in weight;
- paraben preservatives and/or phenoxyethanol in composition of about 1% in weight;
- stabilizing/viscous-making compounds such as cetearyl alcohol and/or PEG-20 stearate comprised between about 5% and 7.5%;
- occlusive and protective moisturizing compounds of the siloxane and xylane type comprised between about 2% to 3% in weight.

The composition as described above for the cosmetic treatment of skin imperfections such as cellulite or adiposeness and the use of a composition as described above for the cutaneous cosmetic treatment of the accumulation of excess liquids which result in water retention at skin level are contemplated.

A method for the cosmetic treatment of the skin that provides to use a composition as described above also comes within the field of the present invention.

According to another feature of the present invention, a method to make a cosmetic composition comprising one fatty part and water to form an oil in water emulsion, comprises the steps defined in claim 9.

In some embodiments of the method of the present invention, the water is of the de-mineralized type, advantageously having an electric conductivity of less than about 5 µS/cm.

In some embodiments of the method of the present invention, the fatty part is comprised between about 6% and 12% in weight, preferably between 7% and 9% in weight.

According to a variant, in the second step a determinate amount of clay is added.

According to a further variant, the first step provides a first cycle of heating the water and a subsequent addition of the viscous-making product which is stirred to obtain a homogenous mixture.

In some embodiments of the method of the present invention, the stirring is performed through the combined action of a turbo emulsor and a planetary stirrer with anchors and blades.

In accordance with one specific embodiment of the invention, the first step provides a first cycle of heating the water up to a temperature comprised between about 70°C and 80°C in the case of xanthan gum, comprised between about 35°C and 45°C for cellulose derived products and a subsequent addition, for example through suction, of the viscous-making product able to reticulate water which is stirred, for example in a turbo emulsor, in order to obtain a homogenous mix thereof.

According to a variant, in the second step, there is a phase in which, after the mixture has been cooled, a check is made to see if there has been a formation of salt crystals.

According to a further variant, the third step there is a second heating cycle of the mixture for the addition of the fatty part, and a subsequent cooling.
In some embodiments of the method of the present invention, the mixture is stirred in order to emulsify the added fatty part, through the combined action of a turbo emulsor and a planetary stirrer with anchors and blades. According to another specific embodiment, in the third step there is a second cycle of heating the mixture, to a temperature comprised between about 70°C and 80° C for xanthan gum, between about 35°C and 45°C for cellulose derived products, for the addition of the fatty part, and subsequent cooling.

Advantageously, in the first step paraben salts are added as a preservative.

A further embodiment of the present invention provides that, after the third step, there is a fourth step in which one or two preservative compounds are added, chosen from the group of ester-phenols and/or alcohol-ethers.

A method as described above which provides, as a propaedeutic and preliminary step, to obtain de-mineralized water at a desired degree of purity and reduced salinity, for example through inverse osmosis, also comes within the field of the present invention

### EXAMPLE 1

### Production of de-mineralized water.

There follows a description of how to de-mineralize and disinfect water, without adding chemical products, by means of the action of ultraviolet rays and electronic control using a microprocessor.

The water entering the plant from the main water system is sent inside a first section of safety filtration at 90 µm, so as to remove any sedimentable corpuscles, bits of rust and particles of sand which may have come from the mains that distributes drinking water.

A section of automatic softening in double column with cationic resins continuously delivered (flow rate of 6,000 liters/an hour, the capacity of each column is equal to 20 m³), removes the hardness salt in order to protect the membranes contained in the osmotic modules from encrustation.

The water treated in this way is then sent inside a carbon clarifier/de-chlorinator filter (carbon filter) with a flow rate of 5,000 liters/an hour, with a de-chlorinating action, in order to carry out a fine filtration and at the same time to remove the residue of any pollutants such as the chloride present in the water when it enters the plant, to stop them reaching the osmotic modules.

The water thus treated, by means of further filtration at 90 µm (to block any possible residual impurities) and filtration at 5 µm, is sent inside the double phase inverse osmosis plant.

The operating requirement of the plant is 4,000 liters/an hour of treated water with a productive yield of 2,000 liters/an hour of osmotized water.

The water passes through a 1 µm membrane filter and then reaches the UV lamp device. Then the water arrives at a 0.2 µm membrane.

At start-up, the biosmosis plant moves the water inside it (re-circulation) for 5 minutes before transferring it to the ring system and/or the storage tanks. The water can be taken from the ring to be used in the processes done in the various sections of the factory.

The value of the conductibility of the water in the ring is monitored by an appropriate device on the osmosis plant which signals the conformity to the specifications set (less than 5 µS/cm, and can even reach about 1µS/cm).

### EXAMPLE 2

### Production of the cosmetic composition

In the method according to the invention an appropriate machine is used, comprising a turbo emulsor of 3,000 liters at about 20°C and a melter for the fatty material.

In a first step, water is loaded into the turbo emulsor, through the water distribution ring served by the osmosis plant described in Example 1.

Then the water is heated by steam generated by boilers located outside the building and which is brought into the jacket of the turbo emulsor. Once the heating has finished, the steam exits from the machine in the form of condensation which is returned to the boilers.

When 75°C is reached, the xanthan gum is aspirated through a tube connected by means of a valve located in the lower part of the machine.

The correct dispersion of the xanthan gum is carefully controlled. The gel which forms is filtered through an appropriate filtering system able to eliminate any possible residual impurity brought by the vegetable gum.

A vacuum is created in the machine so as not to incorporate air and to prevent the semi-worked material from becoming "swollen" and thus not very pleasant. At this point the turbo and the planet (planetary stirring system made up of anchors and blades) are activated for about 20 minutes.

The anchors scrape the wall of the machine taking the semi-worked material upward, and three blades, located more internally and at a different height, take the semi-worked material downward. This allows a very homogenous mixture.

In the first step a small quantity of preservatives are also added, for example paraben salts, such as methyl or propyl-parabenz (Nipagin M) * Isocide MP.

In a second step, salt is added in the same way as for the xanthan gum. Once again the semi-worked material is subjected to filtration in order to guarantee the absence of impurities which may have been brought by the salt.

At this point the product is quickly cooled in the machine (through cooling water introduced into the jacket of the main body), the possible formation of saline crystallizations is checked which, if found, are removed. Otherwise the finished product could have salt crystals which are not aesthetic, and which more importantly would "scratch" the skin of the consumer.

At this step clay is also added, suitable stabilizing and viscous-making compounds, typically Polawax GP200 (cetearyl alcohol) and Emulpharma GP200 (PEG-20 stearate) and moisturizing compounds, occlusive and protective, typically Silicone Fluid 200 (350CS) Miras. DM 350 "BD" (dimethicone, of the siloxene and silane group).

Subsequently the turbo emulsor is heated to 75°C.

In a third step the prime fatty materials are melted in the melter, which is also heated by steam to a temperature of around 80°C. The melter is equipped with a propeller to stir the prime fatty material in the melting phase.

When melting is complete, the oil step in the melter is passed to the water step in the main body of the turbo emulsor through a tube with a filter. Stirring continues with the planet and the turbo for a further 20 minutes, at a constant temperature of 80°C.

After a pre established time and after controlling that emulsion has taken place, the heating is switched off and the cooling process begins by introducing spring water into the jacket of the machine. A constant stirring is kept up with the turbo and the planet at maximum speed.

When the temperature reaches about 50°C the speed of the turbo is lowered in order to facilitate further cooling.

In the case where, instead of xanthan gum, hydroxyethylcellulose is used, the heating cycles described above will have a lower temperature, suitable for this prime material, for example in the range of about 40°C.

In a fourth step, once a temperature of less than 35°C has been reached, a preservative is added, typically Pnenonip*Isocide PN (butylparaben, ethylparaben, isobutylparaben, methylparaben, propylparaben and phenoxyethynol). Functional compounds, perfume and colorants are also added, mixing for 25 minutes with the turbo and the planet at maximum speed, in order to guarantee the perfect homogeneity of the final product.

When the work is completed, a sufficient quantity of the semi-worked product is removed in order to check the parameters of viscosity and pH. Having established the conformity of the semi-worked material, the product is unloaded into containers able to guarantee a high level of hygiene during the period of storage in the warehouse.

There follows a table summing up the percentages in weight of the various components of the cosmetic composition according to one embodiment of the present invention, which also shows the various steps during which the components are added.

| | Component | Percentage weight w/w % |
|---|---|---|
| First step | | |
| | De-mineralized water "BD" | 54.575 - 67.525 |
| | Xanthan gum | 1.9 - 2.5 |
| | Methyl-paraben (Nipagin M) *isocide MP | 0.2 |
| | Propyl-paraben (Nipasol M) *isocide PP | 0.3 |

| Second step | | |
|---|---|---|
| | Ordinary rock salt (NaCl) | 10 - 15 |
| | Clay (Amazonian white clay) | 2 |

| Third step | | |
|---|---|---|
| | Polawax GP200 - Emulpoharma GP200 | 5 - 7.1 |
| | Xalifin 15 | 1 |
| | Karite Butter | 2 |
| | Triglygeride Capril.caprico*Dermol 1*Akomed R | 5 |
| | Silicone Fluid 200 (350CS) Miras. DM350"BD" | 2-3 |

| Fourth step | | |
|---|---|---|
| | Phenoip*Isocide PNB | 1 |
| | Perfume | 1 |
| | Functional | 1 - 5 |
| | Colorants | 0,075 - 0,325 |

## Claims

1. Cosmetic composition comprising one fatty part and water to form an oil in water emulsion, **characterized in that** it also comprises:
- a viscous-making product able to reticulate water comprised between 1% and 3% in weight and chosen from a group including xanthan gum or hydroxyethyl-cellulose;
- sodium chloride comprised between 9% and 30% in weight;
wherein the water is comprised between 50% and 70% in weight.

2. Composition as in claim 1, **characterized in that** sodium chloride is between 10% and 15% in weight.

3. Composition as in claim 1 or 2, **characterized in that** sodium chloride is between 11% and 14% in weight.

4. Composition as in any claim hereinbefore, **characterized in that** the water is of the de-mineralized type, having an electric conductivity of less than 5µS/cm.

5. Composition as in any claim hereinbefore, **characterized in that** it includes a determinate amount of clay.

6. Composition as in any claim hereinbefore, **characterized in that** it comprises one or more preserving compounds chosen from the group of ester-phenols and/or alcohol-ethers and/or paraben salts.

7. Composition as in claim 1, **characterized in that** it comprises:
- de-mineralized water comprised between 54% and 68% in weight;
- said viscous-making product comprised between 1.5% and 3% in weight;
- sodium chloride comprised between 10% and 15% in weight;
- fatty part in composition of 8% in weight;
- paraben preservatives and/or phenoxyethanol in composition of 1% in weight;
- stabilizing/viscous-making compounds comprising cetearyl alcohol and/or PEG-20 stearate comprised between 5% and 7.5%;
- occlusive and protective moisturizing compounds of the siloxane and xylane type comprised between 2% to 3% in weight.

8. Use of a composition as in any claims hereinbefore for topical application to the skin.

9. Method to produce a cosmetic composition comprising one fatty part and water to form an oil in water emulsion, **characterized in that** it comprises the following steps:
- a first step in which a determinate amount of a viscous-making product, able to reticulate water, comprised between 1% and 3% in weight and chosen from a group including xanthan gum or hydroxyethyl-cellulose, is dispersed in water comprised between 50% and 70% in weight;
- a second step in which sodium chloride is added between 9% and 30% in weight;
- a third step in which a determinate amount of fatty material comprised is added in order to create an oil in water emulsion.

10. Method as in claim 9, **characterized in that**, in said second step, sodium chloride is added between 10% and 15% in weight.

11. Method as in claim 9 or 10, **characterized in that**, in said second step, sodium chloride is added between 11% and 14% in weight.

12. Method as in claim 9, 10 or 11, **characterized in that** the first step provides a first cycle of heating the water and a subsequent addition of the viscous-making product which is stirred to obtain a homogenous mixture, wherein the stirring is performed through the combined action of a turbo emulsor and a planetary stirrer with anchors and blades.

13. Method as in claim 12, **characterized in that** in the second step, there is a stage in which, after having cooled the mixture, a control is carried out to check the formation of salt crystals.

14. Method as in any claims from 9 to 13, **characterized in that** in the third step there is a second heating cycle of the mixture for the addition of the fatty part, and a subsequent cooling, wherein the mixture is stirred in order to emulsify the added fatty part, through the combined action of a turbo emulsor and a planetary stirrer with anchors and blades.

15. Method as in any claim from 9 to 14, **characterized in that** in the first step, a fourth step is provided in which one or more preserving compounds are added chosen from the group of paraben salts.

16. Method as in any claim from 9 to 15, **characterized in that** after the third step, a fourth step is provided in which one or more preserving compounds are added, chosen from the group of ester-phenols and/or alcohol-ethers.

## Patentansprüche

1. Kosmetische Zusammensetzung umfassend einen Fettanteil und Wasser, um eine Öl-in-Wasser-Emulsion zu bilden, **dadurch gekennzeichnet, dass** sie auch umfasst:
- ein viskos-machendes Erzeugnis, das Wasser vernetzen kann, das zwischen 1 Gew.-% und 3 Gew.-% enthalten und aus einer Gruppe ausgewählt ist, die Xanthangummi oder Hydroxyethylcellulose umfasst;
- Natriumchlorid, das zwischen 9 Gew.-% und 30 Gew.-% enthalten ist;
worin Wasser zwischen 50 Gew.-% und 70 Gew.-% enthalten ist.

2. Zusammensetzung nach Anspruch 1, **dadurch gekennzeichnet, dass** Natriumchlorid zwischen 10 Gew.-% und 15 Gew.-% vorhanden ist.

3. Zusammensetzung nach Anspruch 1 oder 2, **dadurch gekennzeichnet, dass** Natriumchlorid zwischen 11 Gew.-% und 14 Gew.-% vorhanden ist.

4. Zusammensetzung nach einem der vorhergehenden Ansprüche, **dadurch gekennzeichnet, dass** Wasser vom entmineralisierten Typ ist und eine elektrische Leitfähigkeit von weniger als 5 µS/cm aufweist.

5. Zusammensetzung nach einem der vorhergehenden Ansprüche, **dadurch gekennzeichnet, dass** sie eine bestimmte Menge an Lehm aufweist.

6. Zusammensetzung nach einem der vorhergehenden Ansprüche, **dadurch gekennzeichnet, dass** sie eine oder mehrere konservierende Verbindungen umfasst, die aus der Gruppe von Esterphenolen und/oder Alkoholethern und/oder Parabensalzen ausgewählt sind.

7. Zusammensetzung nach Anspruch 1, **dadurch gekennzeichnet, dass** sie umfasst:
- entmineralisiertes Wasser, das zwischen 54 Gew.-% und 68 Gew.-% enthalten ist;
- das viskos-machende Erzeugnis, das zwischen 1,5 Gew.-% und 3 Gew.-% enthalten ist;
- Natriumchlorid, das zwischen 10 Gew.-% und 15 Gew.-% enthalten ist;
- Fettanteil in der Zusammensetzung von 8 Gew.-%;
- Parabenkonservierungsstoffe und/oder Phenoxyethanol in der Zusammensetzung von 1 Gew.-%;
- stabilisierende/viskos-machende Erzeugnisse, die Cetearylalkohol und/oder PEG-20 Stearat zwischen 5 Gew.-% und 7,5 Gew.-% enthalten;
- okkludierende und schützende befeuchtende Verbindungen des Siloxan- und Xylan-Typs zwischen 2 Gew.-% und 3 Gew.-%.

8. Verwendung einer Zusammensetzung nach einem der vorhergehenden Ansprüche für die topische Auftragung auf die Haut.

9. Verfahren zur Herstellung einer kosmetischen Zusammensetzung umfassend einen Fettanteil und Wasser, um eine Öl-in-Wasser-Emulsion zu bilden, **dadurch gekennzeichnet, dass** es die folgenden Schritte umfasst:
- einen ersten Schritt, in dem eine bestimmte Menge eines viskos-machenden Erzeugnisses, das Wasser vernetzen kann, das zwischen 1 Gew.-% und 3 Gew.-% enthalten und aus einer Gruppe ausgewählt ist, die Xanthangummi oder Hydroxyethylcellulose umfasst, in Wasser, das zwischen 50 Gew.-% und 70 Gew.-% enthalten ist, dispergiert wird;
- einen zweiten Schritt, in dem Natriumchlorid zwischen 9 Gew.-% und 30 Gew.-% hinzugefügt wird;
- einen dritten Schritt, in dem eine bestimmte Menge an fettem Material hinzugefügt wird, um eine Öl-in-Wasser-Emulsion zu schaffen.

10. Verfahren nach Anspruch 9, **dadurch gekennzeichnet, dass** im zweiten Schritt Natriumchlorid zwischen 10 Gew.-% und 15 Gew.-% hinzugefügt wird.

11. Verfahren nach Anspruch 9 oder 10, **dadurch gekennzeichnet, dass** im zweiten Schritt Natriumchlorid zwischen 11 Gew.-% und 14 Gew.-% hinzugefügt wird.

12. Verfahren nach Anspruch 9, 10 oder 11, **dadurch gekennzeichnet, dass** der erste Schritt einen ersten Zyklus von Erhitzen des Wassers und eine nachfolgende Hinzufügung des viskos-machenden Erzeugnisses, das gerührt wird, um eine homogene Mischung zu erhalten, vorsieht, worin das Rühren durch die kombinierte Wirkung eines Turbo-Emulsors und eines Planetenrührers mit Ankern und Blättern durchgeführt wird.

13. Verfahren nach Anspruch 12, **dadurch gekennzeichnet, dass** es im zweiten Schritt eine Stufe gibt, in der nach dem Kühlen der Mischung eine Kontrolle durchgeführt wird, um die Bildung von Salzkristallen zu prüfen.

14. Verfahren nach einem der Ansprüche 9 bis 13, **dadurch gekennzeichnet, dass** es im dritten Schritt einen zweiten Heizzyklus der Mischung für die Hinzufügung des fetten Anteils und eine nachfolgende Kühlung gibt, worin die Mischung gerührt wird, um den hinzugefügten fetten Anteil durch die kombinierte Wirkung eines Turbo-Emulsors und eines Planetenrührers mit Ankern und Blättern zu emulgieren.

15. Verfahren nach einem der Ansprüche 9 bis 14, **dadurch gekennzeichnet, dass** im ersten Schritt ein vierter Schritt vorgesehen ist, in dem eine oder mehrere konservierende Verbindungen hinzugefügt werden, die aus der Gruppe von Parabensalzen ausgewählt sind.

16. Verfahren nach einem der Ansprüche 9 bis 15, **dadurch gekennzeichnet, dass** nach dem dritten Schritt ein vierter Schritt vorgesehen ist, in dem eine oder mehrere konservierende Verbindungen hinzugefügt werden, die aus der Gruppe von Esterphenolen und/oder Alkoholethern ausgewählt sind.

## Revendications

1. Composition cosmétique comprenant une partie grasse et de l'eau pour former une émulsion huile dans eau, **caractérisée en ce qu'**elle comprend également :
- un produit générateur de viscosité apte à réticuler l'eau, compris entre 1 % et 3 % en poids et choisi dans le groupe comprenant la gomme xanthane ou l'hydroxyéthylcellulose ;
- du chlorure de sodium compris entre 9 % et 30 % en poids ;
l'eau étant comprise entre 50% et 70 % en poids.

2. Composition selon la revendication 1, **caractérisée en ce que** le chlorure de sodium est compris entre 10 % et 15 % en poids.

3. Composition selon la revendication 1 ou la revendication 2, **caractérisée en ce que** le chlorure de sodium est compris entre 11 % et 14 % en poids.

4. Composition selon l'une quelconque des revendications précédentes, **caractérisée en ce que** l'eau est du type déminéralisé, ayant une conductivité électrique inférieure à 5µS/cm.

5. Composition selon l'une quelconque des revendications précédentes, **caractérisée en ce qu'**elle comprend une quantité déterminée d'argile.

6. Composition selon l'une quelconque des revendications précédentes, **caractérisée en ce qu'**elle comprend un ou plusieurs composés de conservation choisis dans le groupe des esters-phénols et/ou alcool-éthers et/ou sels de parabène.

7. Composition selon la revendication 1, **caractérisée en ce qu'**elle comprend :
- de l'eau déminéralisée, comprise entre 54 % et 68 % en poids ;
- ledit produit générateur de viscosité, compris entre 1,5 % et 3 % en poids ;
- du chlorure de sodium, compris entre 10 % et 15 % en poids ;
- une partie grasse dans la composition, à hauteur de 8 % en poids ;
- des conservateurs au parabène et/ou du phénoxyéthanol, à hauteur de 1 % en poids de la composition ;
- des composés stabilisants/générateurs de viscosité contenant de l'alcool cétéarylique et/ou du stéarate de PEG-20 compris entre 5 % et 7,5 % ;
- des composés hydratants occlusifs et protecteurs de type siloxane et xylane compris entre 2 % et 3 % en poids.

8. Utilisation d'une composition selon l'une quelconque des revendications précédentes pour une application topique sur la peau.

9. Procédé de fabrication d'une composition cosmétique comprenant une partie grasse et de l'eau pour former une émulsion huile dans eau, **caractérisé en ce qu'**il comprend les étapes suivantes :
- une première étape dans laquelle une quantité déterminée d'un produit générateur de viscosité, apte à réticuler l'eau, comprise entre 1 % et 3 % en poids et choisie dans un groupe comprenant la gomme xanthane ou l'hydroxyéthylcellulose, est dispersée dans de l'eau comprise entre 50 % et 70 % en poids ;
- une deuxième étape dans laquelle du chlorure de sodium est ajouté entre 9 % et 30 % en poids ;
- une troisième étape dans laquelle une quantité déterminée de matière grasse contenue est ajoutée afin de créer une émulsion huile dans eau.

10. Procédé selon la revendication 9, **caractérisé en ce que**, dans ladite deuxième étape, du chlorure de sodium est ajouté, entre 10 % et 15 % en poids.

11. Procédé selon la revendication 9 ou la revendication 10, **caractérisé en ce que**, dans ladite deuxième étape, du chlorure de sodium est ajouté, entre 11 % et 14 % en poids.

12. Procédé selon la revendication 9, 10 ou 11, **caractérisé en ce que** la première étape inclut un premier cycle de chauffage de l'eau et une addition ultérieure du produit générateur de viscosité qui est agité de façon à obtenir un mélange homogène, l'agitation étant réalisée par l'action combinée d'un turbo- émulsificateur et d'un mélangeur planétaire à ancres et lames.

13. Procédé selon la revendication 12, **caractérisé en ce que** dans la deuxième étape, il y a une étape dans laquelle, après avoir refroidi le mélange, un contrôle est effectué pour vérifier la formation de cristaux de sel.

14. Procédé comme selon l'une quelconque des revendications 9 à 13, **caractérisé en ce que** dans la troisième étape, il y a un deuxième cycle de chauffage du mélange pour l'addition de la partie grasse, et un refroidissement subséquent, le mélange étant agité afin d'émulsionner la partie grasse ajoutée, par l'action combinée d'un turbo-émulsificateur et d'un mélangeur planétaire à ancres et lames.

15. Procédé selon l'une quelconque des revendications 9 à 14, **caractérisé en ce que** dans la première étape, il est prévu une quatrième étape dans laquelle un ou plusieurs composés de conservation sont ajoutés, choisis dans le groupe des sels de parabène.

16. Procédé selon l'une quelconque des revendications 9 à 15, **caractérisé en ce qu'**après la troisième étape, il est prévu une quatrième étape dans laquelle un ou plusieurs composés de conservation sont ajoutés, choisis dans le groupe des esters-phénols et/ou des éthers d'alcool.
